# EUROPEAN PATENT SPECIFICATION

(11) **EP 2 033 667 B1**
(45) Date of publication and mention of the grant of the patent: **13.02.2013**
(21) Application number: 08010581.0
(22) Date of filing: 15.04.1998
(51) Int. Cl.: A61F 2/06, A61L 31/00, A61L 31/16, A61L 31/14, A61L 31/02

(54) **Porous medicated stent**
Poröser medizinischer Stent
Endoprothèse médicamenteuse poreuse

(30) Priority: 15.04.1997 US 842660
(43) Date of publication of application: 11.03.2009
(62) Divisional of application: 05002387.8
(73) Proprietor: Abbott Cardiovascular Systems Inc., Santa Clara, CA 95054 (US)
(72) Inventor: Yan, John Y., California 95032 (US)
(74) Representative: McLeish, Nicholas Alistair Maxwell

(56) References cited:
- EP-A- 0 671 165
- EP-A- 0 734 721
- CA-A1- 2 178 541
- US-A- 4 374 669

## Description

### BACKGROUND OF THE INVENTION

### Field of the Invention:

This invention generally relates to a medicated prosthesis or implant. More particularly, the invention relates to a medicated intra-vascular prosthesis, such as a stent, that is radially expandable in the vasculature of a patient and delivers a therapeutic agent to the site of the implantation.

### Description of Related Art:

Stents are generally cylindrically-shaped prosthetic implants which function to hold open and sometimes expand a segment of a blood vessel or other anatomical lumen. They are particularly suitable for supporting and preventing a torn or injured arterial lining from occluding a fluid passageway. Intravascular stents increasingly are useful for treatment of coronary artery stenoses, and for reducing the likelihood of the development of restenosis or closure after balloon angioplasty.

The success of a stent can be assessed by evaluating a number of factors, such as thrombosis; neointimal hyperplasia, smooth muscle cell migration and proliferation following implantation of the stent; injury to the artery wall; overall loss of luminal patency; stent diameter in vivo; thickness of the stent; and leukocyte adhesion to the luminal lining of stented arteries. However, the chief areas of concern are early subacute thrombosis, and eventual restenosis of the blood vessel due to intimal hyperplasia.

Therapeutic pharmacological agents have been developed to improve successful placement of the stent and are delivered to the site of stent implantation. Stents that are of a common metallic structure were previously unable to deliver localized therapeutic pharmacological agents to a blood vessel at the location being treated with the stent. There are polymeric materials that can be loaded with therapeutic agents including drugs or other pharmacological treatments which agents then can be released for drug delivery. However, these polymeric materials may not fulfill the structural and mechanical requirements of a stent, especially when the polymeric materials are loaded with a drug, since drug loading of a polymeric material can significantly reduce the structural and mechanical properties of the polymeric material.

CA-A-2,178,541 discloses a coronary stent that includes a structure adapted for introduction into the vascular system; at least one layer of a bioactive material posited on one surface of the stent structure; and at least one porous layer posited over the bioactive material layer and the bioactive-material-free stent surface. The porous layer is comprised of a polymer applied preferably by vapour or plasma deposition and provides a controlled release of the bioactive material.

By contrast US-A-4,374,669 describes a cardiovascular prosthetic device or implant comprising a porous surface and a network of interconnected interstitial pores below the surface in fluid flow communication with the surface pores. Tissue forms a smooth thin adherent coating of self-determining thickness on the porous surface making it resistant to the formation of the blood clots normally associated with the presence of foreign bodies in the blood stream.

It is known in the art to coat a metallic stent with a polymeric material and to load the polymeric material with a drug. Alternatively, stents of polymeric materials have been reinforced with metal structure. These stent designs have the strength necessary to hold open the lumen of the vessel because of the reinforcement contributed by the metal. Stents made of both polymeric material and metal have a larger radial profile because the volume occupied by the metal portion of the stent cannot absorb and retain drugs. Reducing the profile of a stent is desirable because doing so increases the in vivo diameter of the lumen created by the stent. Thus it is desirable to configure a metallic stent to deliver drugs to the blood vessel walls without substantially increasing the profile of the stent.

### SUMMARY OF THE INVENTION

According to the present invention there is provided s stent as claimed in the appended claims.

Certain preferred embodiments of the present invention provide for an implantable prosthesis that is made of metal and has pores or porous cavities in the metallic portion of the prosthesis so that therapeutic agents such as drugs can be loaded directly into the pores without substantially weakening the structural and mechanical characteristics of the prosthesis. The stent of one embodiment of the present invention can be implanted at a specific site of vascular injury, such as can result from balloon angioplasty or de novo lesions of a therosclerotic disease. The drugs in the pores of the stent can treat restenosis and tissue inflammation, and promote endothelialization or any other disease or condition that might detract from the success of a stent implantation.

The pores of the stent are formed by sintering the stent material from metallic particles, filaments, fibers or other materials. The stent can be formed from a sintered wire that is coiled or otherwise is formed into a stent. The stent can be formed from a sintered cylindrical tube or sintered metal sheet which can be laser-cut or chemically etched into an expandable stent structure.

Additionally, the porosity of the stent metal can be increased by using particles that are not generally spherical such as fibrous particles, filaments or wires. In one embodiment of the invention, the interwoven fibers and filaments also can be sintered after they have been woven into the desired shape.

In one embodiment of the present invention, the stent is formed from a metal wire or strut that is comprised of a first layer of particles, oriented along a first axis, which forms a core, and an outer layer of particles that are arranged radially outward from the inner layer of particles. The particles in the outer layer have a smaller diameter than the particles in the inner layer. The core permits a greater volume of drugs in the center of the stent. The smaller diameter particles on the outside control the rate at which drugs are released into the walls of the vessel. The larger diameter particles result in a layer of greater porosity which thus is capable of carrying a higher volume of medication.

In another embodiment it may be desirable to form a stent that has a solid core and a porous outer section. This can be accomplished by sintering particles to a solid, non-porous, metal wire. A stent so configured has a solid core which reinforces the structure of the stent and thereby adds strength to the device. The porous particles sintered to the surface of the stent absorb drugs for delivery.

In one embodiment according to the present invention, the stent is formed from a sheet or tube of sintered metal. The sheet or tube is cut according to a pattern that allows the stent to be expanded and thus deployed into the vasculature. The stent pattern of this embodiment can be stenciled onto the sheet or tube of sintered metal and then may be cut by laser-cutting the sheet into the desired shape. Alternatively, the stent can be chemically etched into its desired shape.

According to another embodiment of the invention, the stent receives a coating on the surface of the stent. In certain applications, it is desirable that the coating be a biopolymer and in other applications, the coating preferably is a synthetic polymer or a hydrogel. The coating also can be a heparin coating that is affixed to the surface of the stent through ionic bonding, end-point attaching or photolinking the heparin.

According to one embodiment of the invention, the coating preferably is permeable to the medication. The permeability of the coating should be selected to release the medication in the stent at a desired rate.

In another embodiment of the present invention, a bioabsorbable coating is applied to the stent. This coating is dissolved by the body fluids. Further, it is desirable in certain applications to load medication into the coating applied to the stent. According to one application, the coating preferably is loaded with the same drug or medication that is loaded into the stent. In another embodiment, the coating is loaded with a medication that is different than that which is carried by the stent. In this configuration, the two medications are released sequentially.

The method of using the stent of the present invention comprises providing a porous prosthesis, loading a drug into the pores of the prosthesis, positioning the prosthesis in an appropriate site and affixing the prosthesis to the site. In another arrangement, the method further includes the step of applying a coating to the stent after the step of loading the drug.

These and other features of the present invention will become apparent from the following more detailed description, when taken in conjunction with the accompanying drawings which illustrate, by way of example, the principles of the present invention.

### BRIEF DESCRIPTION OF THE DRAWINGS

FIGURE 1 is a longitudinal sectional view of a blood vessel with a stent manufactured according to one embodiment of the present invention.
FIG. 2 is a porous stent wire or strut in a partially magnified, partially cut-away perspective, manufactured according to one embodiment of the present invention.
FIG. 3 is a magnified, cross-sectional view of un-sintered, packed particle.
FIG. 4 is a porous stent wire or strut in a partially magnified, partially cut-away perspective, manufactured according to one embodiment of the present invention.
FIG. 5 is a porous stent wire or strut in a partially magnified, partially cut-away perspective, manufactured according to one embodiment of the present invention.
FIG. 6 is a cross-sectional view of a stent wire or strut manufactured according to one embodiment of the present invention.
FIG. 7 is a cross-sectional view of a stent wire or strut: formed from sintered metal particles surrounding a solid core.
FIG. 8 is a sheet of sintered stent manufactured according to one embodiment of the present invention.
FIG. 9 is a stent formed from a sheet of sintered metal according to one embodiment of the present invention.
FIG. 10 is a cross-sectional, partially cut-away view of a sheet of sintered metal that has particles that are sintered to both sides of the sheet.
FIG. 11 is a cross-sectional view of a stent wire or strut having a coating applied to the sintered metal.
FIG. 12 is a cross-sectional view, partially cut- away of a sheet of sintered metal manufactured according to the principles of one embodiment of the present invention.

### DETAILED DESCRIPTION OF THE PREFERRED EMBODIMENTS

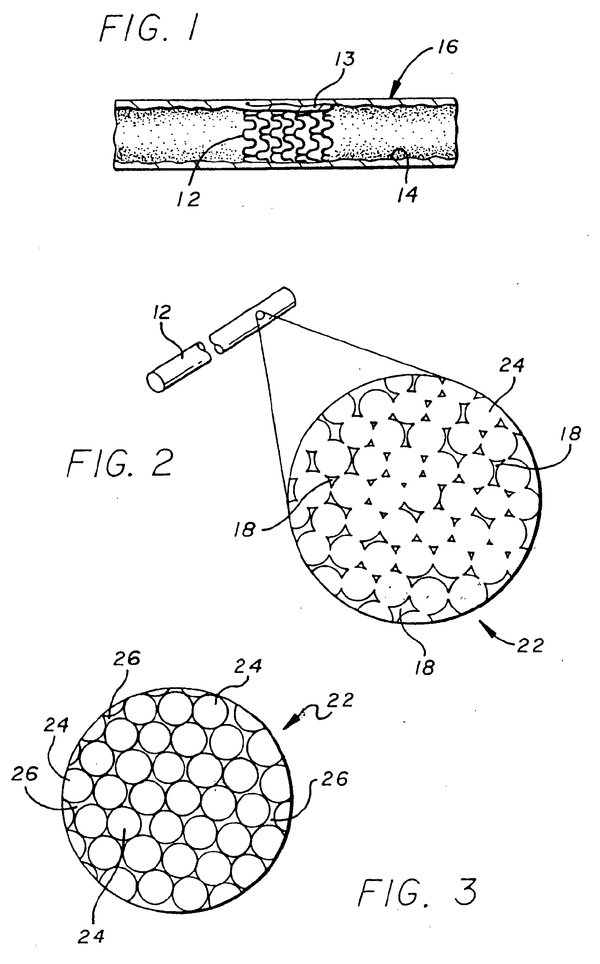

Referring to FIGURE 1, the prosthesis of one embodiment is a porous stent 12 that is radially expandable against the walls 14 of a vessel 16. A therapeutic agent is loaded into the pores 18 (See FIG. 2) of the stent. When placed in the vasculature, the therapeutic agent is delivered to the tissue that comes into contact with the stent. The stent of one preferred embodiment is formed of a stent wire that is porous. An example of a porous stent wire is a sintered metal wire. FIG. 2 illustrates a partial microscopic view of a sintered wire that is suitable for use in one embodiment of the present invention. The wire is porous and has several pores 18. The cavities preferably range in size between 0.01 and 20 µm.

The metal is made porous by the process of sintering metal. Sintering is a process of fabrication where particles are bonded together to form a coherent mass without entirely melting the particles. Particles are pressed together or molded into a desired shape. A considerable amount of pressure first is applied to press the particles together. Then the metal is heated to temperatures slightly below the melting point of the metal. Without entirely melting, the particles bond to each other. Space remains between the lattice of the particles and this space defines the pores 18.

The formation of sintered metal is illustrated with reference to FIG. 3 and continued reference to FIG. 2. FIG. 3 is a microscopic view of a packed lattice 22 of metallic particles 24. Gaps 26 exist between each particle despite the fact that the particles are pressurized and are in contact with adjacent particles. Particles preferably are sized between 0.02 µm and 20 µm in diameter. Prior to heating, there are no chemical bonds formed between the individual particles. When the metal is heated to slightly below the melting point of the metal, the particles bond with neighboring particles. The gaps in the packed lattice form pores 18 when the particles are sintered. Thus in FIG. 2, the metal stent wire formed by the process of sintering has pores 18 extending throughout the entire wire, thereby interconnecting the cavities. The cavities then can be filled with a therapeutic agent as hereinafter described. The appropriate pressure and temperature of sintering a particular metal is specific to that particular metal. One skilled in the art of metal fabrication would understand how to sinter any given metal or alloy.

For each of the embodiments, the metal stent material member can be a suitable metal such as stainless steel, tantalum, nickel-titanium alloy, platinum-iridium alloy, molybdenum-rhenium alloy, gold, magnesium, combinations thereof, although other similar materials also may be suitable. The metal can be modified to exhibit different hardnesses, and thus varying stiffnesses, by well known annealing and manufacturing processes.

One of the factors to be considered when making a stent according to the present invention is the porosity of the metal. Porosity is the total volume of pores in the sintered metal divided by the total volume of the metal. Porosity determines the amount of a therapeutic agent that can be loaded into a stent of predetermined dimensions. High porosity means that a stent can deliver more therapeutic agents or have a narrower profile because it is less dense. High porosity, according to some embodiments of the present invention, adversely affects the strength and elasticity of a metal. Consequently, there is an ongoing tradeoff between stent strength, on the one hand, and stent profile and stent load capacity on the other hand.

Pore size is a function of the size of the particles which create the gaps that establish the pores. As illustrated in FIG. 3, the particles 24 may be generally spherical. The size of each pore 18 is proportional to particle size, particularly with generally spherical particles. When the particles 24 are not of uniform size, smaller particles tend to fill the gaps between larger particles. Thus, the porosity of the metal formed with such particles is less predictable than when more equally-sized particles are used. General uniformity of pore size also is important to ensure that drugs are dispersed evenly throughout the stent. A generally uniform distribution of pores insures that the tissue in contact with the stent will receive an evenly distributed dose of a therapeutic agent.

There are several types of drugs that currently are administered at the site that a stent is placed in the vessel. Examples of therapeutic drugs, or agents that can be combined with the particle layers, include antiplatelets, anticoagulants, antifibrins, antithrombins and antiproliferatives. Examples of antiplatelets, anticoagulants, antifibrins and antithrombins include but are not limited to sodium heparin, low molecular weight heparin, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antibody, recombinant hirudin, thrombin inhibitor (available from Biogen), and an antiplatelet drug sold under the trademark "7E-3B" by Centorcor, Inc. Examples of cytostatic or antiproliferative agents include angiopeptin, a somatostatin analogue; angiotensin-converting enzyme inhibitors, such as those manufactured under the trademarks "Captopril" (by Squibb Corp.), "Cilazapril" (by Hoffman-LaRoche, Inc.) and "Lisinopril" (by Merck & Co., Inc.); calcium channel blockers such as nifedipine; colchicine; fibroblast growth factor (FGF) antagonists; fish oils, such as omega 3 fatty acids; cholesterol-lowering drugs such as inhibitors of HMG-CoA reductase, one of which is sold under the trademark "Lovastatin" by Merck & Co., Inc.; methotrexate, monoclonal phosphodiesterase inhibitors, prostaglandin inhibitor (available from Glaxo Wellcome, Inc., PDGF antagonists such as seramin and triazolopyrimidine, serotonin blockers, steroids, thioprotease inhibitors, and nitric oxide. Other therapeutic drugs which may be appropriate include alpha-interferon and genetically-engineered epithelial cells, for example.

The foregoing therapeutic agents have been used to prevent or to treat restenosis, and each is identified by way of example and not by limitation, as other therapeutic drugs may be developed which equally are applicable for use with the present invention. Using such therapeutic agents to treat vessels or body lumens is known in the art, as is the calculation of dosages, dosage rates and appropriate duration of treatment.

The therapeutic agent of one embodiment preferably is in liquid form and is loaded into a stent by immersing the stent in a medicated solution. The therapeutic agent may be dissolved in a solvent or suspended in a liquid mixture. If a suspension is used, it is important that the pore size of the stent is considerably larger than the suspended particles of the therapeutic agent. An average pore size that is more than ten (10) times the particle size of a suspended therapeutic agent is suitable. After the stent is immersed in the medicated solution, the therapeutic agent is absorbed into the pores of the stent. The loaded stent then can be removed from the solution and implanted into the vasculature of a patient. Optionally, the loading of the therapeutic agent into the stent can be facilitated by applying pressure to the fluid in which the agent is dissolved or suspended. The applied pressure will aid the passage of medicated fluid into the pores of the stent. This technique might be likened to the physical process of forcing a fluid through the pores of a filter.

Once loaded into the stent the therapeutic agent remains in place by reason of the surface tension between the outer surfaces of the particles that form the pores 18 and the particles of the therapeutic agent. As shown in FIG. 1, the loaded or medicated porous stent 12 then is deployed to the site of an arterial closure 13 and is expanded. The expanded stent engages the walls 14 of the vessel 16 to maintain the patency of the vessel. Once in the vessel and as is illustrated in FIG. 2, the therapeutic agent disseminates from the pores 18 and is absorbed into the tissue of the walls of the vessel that are in contact with the stent.

Among the advantages of a stent embodying the present invention over prior art medicated stents are its profile and strength. Metal, including sintered metal, is stronger than synthetic materials, such as polymer blends, which are capable of being loaded with a therapeutic agent. Thus, in order for a medicated stent to deliver an appropriate amount of a therapeutic agent and structurally maintain vessel patency, the radial profile of the stent must be substantially larger than that of metal stents. This is true whether or not a metal stent is coated with a polymeric material to carry a therapeutic agent, or if the stent is made entirely of a plastic material.

Sintered metal has strength and elasticity that is comparable to non-sintered metal. Sintered metal further has the added feature of porosity. Consequently, a sintered metal stent can be manufactured with a profile that is substantially comparable to that of a conventional metal stent, and a therapeutic agent can be loaded into the pores and delivered to the site of stent implantation without the aid of medicated polymer coatings.

Additionally, many synthetic materials, including materials that are bioabsorbable, can cause inflammation of the tissue. A medicated metal stent having a therapeutic agent loaded directly into the pores of the stent likely will be less apt to cause irritation at the site of implantation.

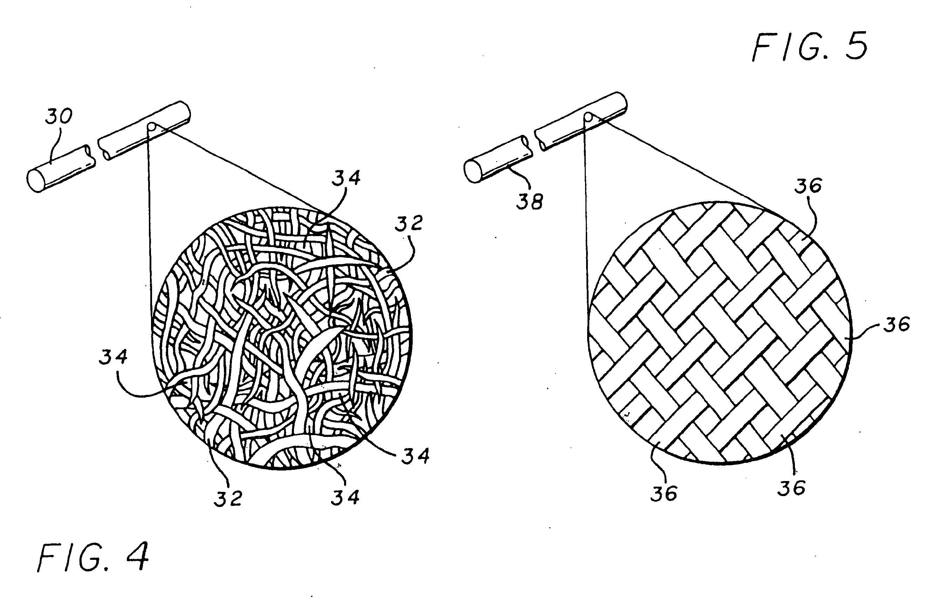

FIG.4 illustrates a stent wire 30 constructed according to an embodiment of the invention. The stent is formed of elongated particles 32, i.e., filaments and fibers. When generally spherically-shaped particles of metal are used to compose sintered metal, the resultant porosity typically is in the range of five to thirty percent. When the particles are elongated, these filaments or fibers can result in a porosity of greater than thirty percent when sintered. The technique of fabricating a stent with elongated filaments or fibers is similar to the method described above for spherical particles or powders. The filaments or fibers are molded and pressurized. Then the fibers are heated to a temperature just below the melting point of the metal.

A stent made of metal filaments or fibers 32 rather than spherical particles (such as those illustrated in FIG. 2) exhibits greater porosity because of the irregular shape of the particles. The particles can be packed less densely than uniformly-shaped particles but contact between the irregularly-shaped particles nevertheless can be maintained to allow sintering. Thus, the void space or pores 34 in the sintered metal tend to be larger than the pores 18 that result from spherical particle sintering.

The strength of a stent wire 30 using filaments in FIG. 4 is improved because, upon sintering, the individual strands have a greater surface-area-to-volume ratio and will contact more neighboring strands than will spherical particles. Thus, each filament or fiber will have a greater surface area on which to bond with adjacent filaments or fibers. A matrix of overlapping filaments or fibers thus is formed exhibiting greater porosity and stronger inter-particle bonding.

In yet another embodiment, wire fibers 36 are woven or twined into a structure 38 as illustrated in FIG. 5. The individual strands cooperate in a synergistic manner to reinforce the strength of the wire. Additionally, the wire fibers can be woven into the form of a sintered metal sheet having improved and reinforced strength or into a sintered metal tube. Other combinations of particle size and shape can be employed to form a stent wire having different characteristics.

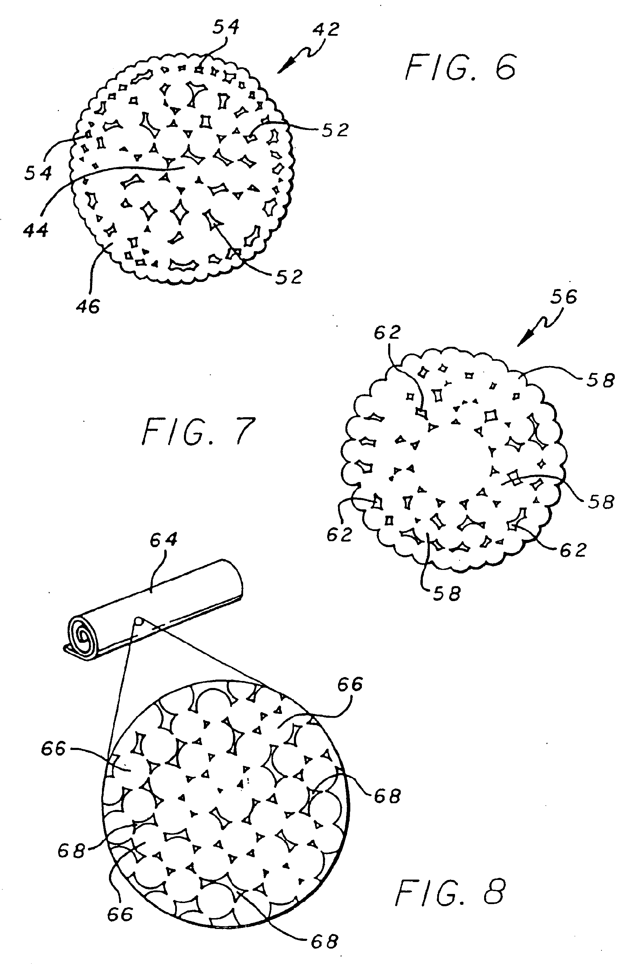

As illustrated in FIG. 6, the stent wire 42 is formed of an inner core 44 and an outer layer 46 of sintered particles. The outer layer is formed from particles having a different diameter than the diameter of the particles that form the inner core. For example, the core of the metal is formed of particles that have a diameter in the range of 10-20 µm. Surrounding the core are particles that have a diameter in the range of: 2-4 µm. The larger particles create a core having larger pores 52. This results in higher porosity and thus a higher load capacity. The smaller particles on the outer layer form smaller pores 54 which reduce the rate of diffusion of drugs into the tissue of the vessel.

When a therapeutic agent is loaded into a stent formed of the stent wire 42 illustrated in FIG. 6, a larger volume can be stored in the larger pores 52 at the core 44 of the stent wire. Once the stent is placed into the vessel, the therapeutic agent in the stent wire is delivered at a rate that is determined by the smaller pores 54 in the outer layer 46 of the stent wire. Such a structure is expected to be capable of storing a large amount of therapeutic agent at the core and of delivering the therapeutic agent at a slower rate than would be accomplished if the pores of the stent wire were of more uniform size. Consequently, this design is appropriate when long-term drug therapy at a low dosage rate is desired.

FIG. 7 shows a stent wire 56 formed from sintered particles 58. The pores 62 formed between the sintered metal particles surrounding the solid core retain the therapeutic agent. The overall porosity of a stent having a solid core and porous outer layer is much less than that of a stent wire having similar proportions but which is composed entirely of sintered particles. However, the solid core reinforces the tensile strength and elasticity of the metal stent and is considerably stronger than a uniform-particle sintered stent. Thus, it is desirable to use a sintered stent with a solid core for applications where maximum tensile strength and elasticity is desirable and only a relatively small amount of therapeutic agent is needed.

The sintered metal stent of the present invention can be made of material formed in the spherically-shaped or filament-like particles discussed previously. For example, the stent can be formed of a sheet of sintered metal 64 as shown in FIG. 8 or of a sintered metal tube. By way of example, metal particles 66 are arranged and pressurized into a sheet. The sheet then is heated to a temperature below the melting point of the particles as described previously. The sheet of sintered metal is porous and has a plurality of pores 68.

The same principles that apply to porosity and pore size of a wire apply equally to a sintered stent that is formed into a sheet or tube. The advantage of forming the stent from a sheet of metal is that the stent is radially expandable without placing a great deal of strain on the metal lattice when it is expanded. A sheet or tube of sintered metal can be cut in the desired shape to form the metal structural member with a laser, such as a continuous CO₂ laser, a pulsed YAG laser, or an excimer laser, for example, or alternatively, by chemical etching or stamping. When cut from a flat sheet, the stent then is rolled into a cylindrical configuration and is laser welded along the longitudinal edges.

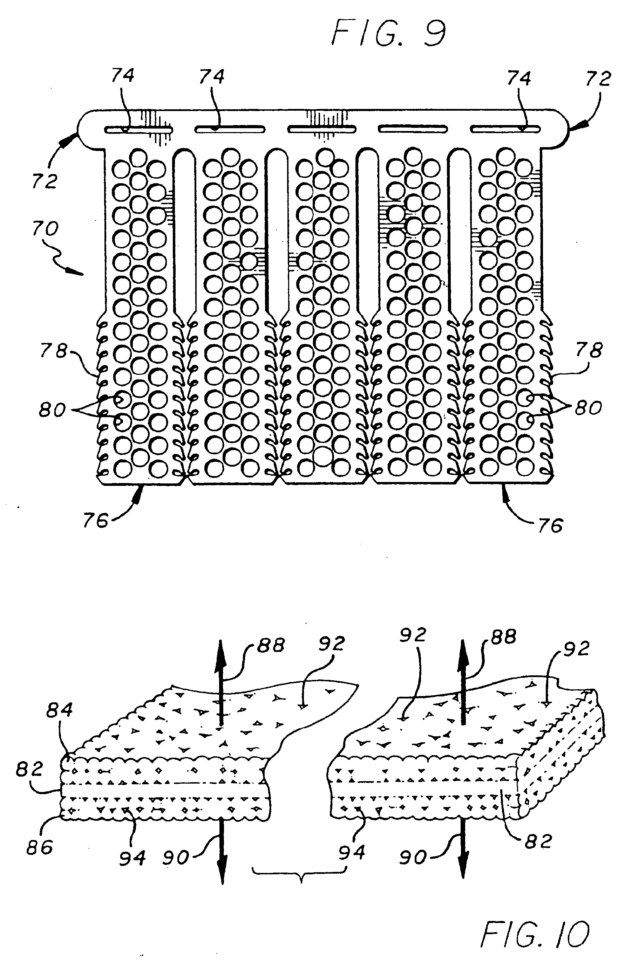

The stent can be formed into a particular pattern known in the art for stents formed from metal sheets. One such pattern is a rolled locking design and is illustrated in FIG. 9. The sheet is etched into a stent configuration 70 that has a head portion 72 that includes one Or more slots 74 for receipt of a number of tail sections 76 that correspond to each slot. The tail sections are received into the slots so as to form a cylindrical loop. Each tail section includes a plurality of teeth 78 that are adapted to cooperatively engage the slot of the head portion. When the teeth engage the slot, the tail sections are retained in place, holding the stent configuration in an expanded state. Additionally, holes 80 are formed throughout the stent to reduce the metal-to-air ratio of the stent. The less metal that is in contact with the wall 14 of the vessel 16, the more likely the stent is to be compatible with the blood.

Prior to deployment, the tail sections are coiled into a retracted position in a "jelly-roll" fashion. Each tail section then is threaded through each corresponding slot and wound. The stent configuration is expanded by a balloon according to principles that are well known in the art for delivering and implanting stents. As the stent configuration 70 is expanded by a balloon during deployment, it unwinds and the teeth 78 lock into the slots 74 at a desired radial diameter to prevent the stent from returning to its original, retracted state.

A benefit of the coiled stent shown in FIG. 9 is that the stent 70 can be etched to have a minimal surface area that comes in contact with the walls of the vessel. This may be an important feature when it is desired to cover the entire area of the walls of a blood vessel with a therapeutic agent because the coiled sheet metal stent can be configured to maintain maximum surface area contact with the wall of the blood vessel in contrast to wire stents.

With reference to FIG. 10, there is shown a sheet that has particles that are sintered to both sides 84 and 86 of a metal sheet 82. The stent of FIG. 10 is similar in structure to the stent wire of FIG. 7 in that it has a solid core and particles sintered to the core forming a porous outer layer. The solid core reinforces the strength of the metal. The metal sheet also provides a barrier through which a therapeutic agent cannot pass. Thus, a therapeutic agent loaded into the pores 92 on the top side 84 of the sheet permeates in a first direction 88 outward from the solid core. A therapeutic agent loaded into the pores 94 on the bottom side 86 of the solid wire permeates only in a second direction 90 which is opposite to the direction of the therapeutic agent loaded into the pores on the top side.

When a stent as shown in FIG. 10 is looped into a cylindrical formation and placed into a vessel, only the top side 84, which is directed radially outward, engages the walls of the vessel. The bottom side 86 faces radially inward and does not come in contact with the walls of the vessel. Thus, if it is desired, a first therapeutic agent can be loaded into the top side to treat the tissue in the wall of the vessel. A second therapeutic agent can be loaded into the bottom side to prevent coagulation of the blood flowing in the vessel. Additionally, the stent can be formed so that particles are sintered only to one side of the stent. A therapeutic agent is loaded into the sintered metal on the porous side of the stent. When a stent is formed with only one porous side, that side can be oriented radially outward to deliver a therapeutic agent to the tissue in the wall of the stent.

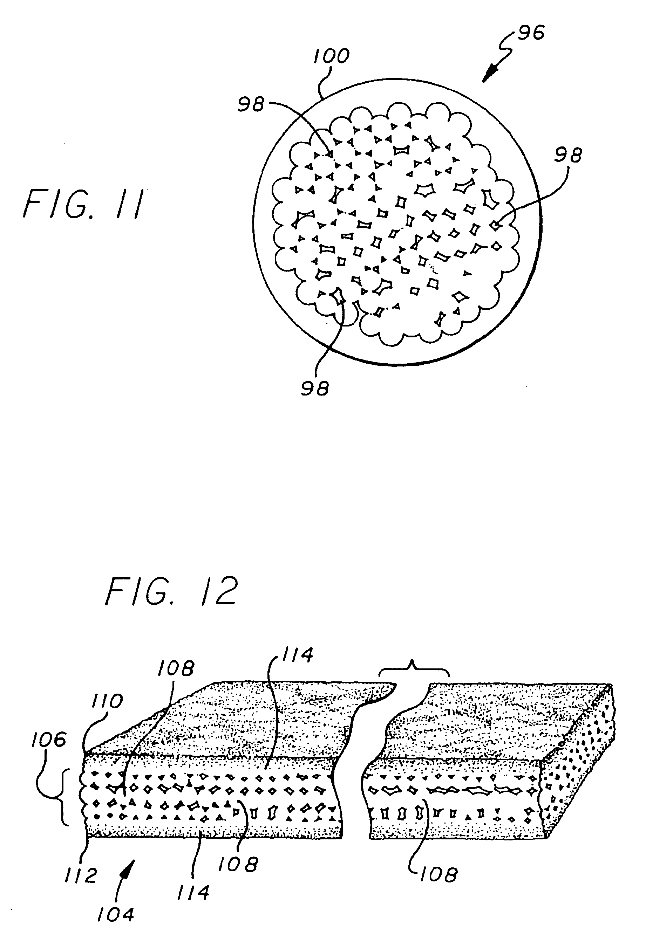

FIG. 11 illustrates a cross-sectional view of a stent wire according to another arrangement. The sheet has a plurality of porous cavities or pores 98. A therapeutic agent is loaded into the pores of the sintered metal. Then, a coating 100 is applied to the sintered metal. The coating may be used for several purposes as described hereinafter.

With reference to FIG. 12, another embodiment of the invention is shown wherein the stent is formed of a sintered sheet 104 of metal having core 106 formed of larger-diameter particles 108 that form larger pores as a result of sintering. The core layer 106 is sandwiched between two layers 110 and 112 formed of smaller-diameter particles 114 or particles that result in the formation of smaller pores. Such a sheet is formed by orienting the middle or core layer 106 of larger-diameter particles 108 along a plane. A top layer of the smaller-diameter particles is arranged in a plane parallel to and above the core layer. A bottom layer of particles are arranged in a plane parallel to and below the core layer. The three layers then are pressed together and sintered into a single sheet. The sheet then can be cut or etched into a stent configuration. While one of the benefits of certain embodiments is to provide a stent that does not require a coating for the purpose of delivering a therapeutic agent to the blood vessel, the application of a coating after a therapeutic agent has been loaded into the pores of the sintered metal does not defeat the utility of the present invention. For example, when a therapeutic agent is loaded into the pores of the stent and also into a polymeric coating, the profile of the polymeric coating can be reduced. When such a polymeric coating is applied, a larger dosage of a therapeutic agent can be delivered to the site of stent implantation. Additional benefits can be obtained by loading a stent with a therapeutic agent in the pores of the metal and by then further applying a polymeric coating to the stent. Even if a polymeric coating is applied to the stent, the principles of reducing profile and reinforcing the stent still are realizable because a greater volume of therapeutic agent can be delivered by a polymeric-coated sintered stent than by a coated, all-polymeric stent with comparable dimensions.

The polymeric material that coats a sintered metal stent preferably comprises a biodegradable, bioabsorbable polymeric film that is capable of being loaded with and subsequently releasing therapeutic drugs. The polymeric coatings preferably include, but are not limited to, polycaprolactone (PCL), poly-DL-lactic acid (DL-PLA) and poly-L-lactic acid (L-PLA) or lactide. Other biodegradable, bioabsorbable polymers such as polyorthoesters, polyiminocarbonates, aliphatic polycarbonates, and polyphosphazenes also may be suitable, and other non-degradable polymers capable of carrying and delivering therapeutic drugs might be appropriate as well. Examples of non-degradable synthetic polymers are those sold under the trade names "Parylene" and "Parylast" by Advanced Surface Technology of Billerica, MA, polyurethane, polyethylene, polyethylene teraphthalate, ethylene vinyl acetate, silicone and polyethylene oxide (PEO). The polymeric layer, according to one embodiment, is loaded with a pharmacologic agent for use in localized drug therapy. As used in this description, the terms biodegradable, bioabsorbable, reabsorbable, degradable, and absorbable collectively are meant to encompass materials that are broken down and gradually absorbed or eliminated by the body, whether these processes are due to hydrolysis, metabolic processes, or to bulk or surface erosion. In each of the foregoing embodiments, one polymeric layer preferably is about 0.051 millimeters (0.002 inches) thick.

The thin polymeric films used to coat the stent preferably first are intermixed with the drug or drugs to be delivered, and then typically are laminated or solvent cast to the surface of the metal structural member. Lamination processing methods and temperatures can vary widely depending on the polymers used and the temperature sensitivity of the loaded drugs. Alternatively, the metal structure of the stent can be encapsulated in the layers of polymeric material by solvent casting, melt processing, insert molding, and dip coating.

In one embodiment of the present invention, the membrane is bioabsorbable, but no therapeutic agent is loaded into the polymer. The coating dissolves after implantation and this delays the time that a therapeutic agent is released into the vasculature of a patient. The thickness of the coating, as well as the rate at which the coating is bioabsorbed, determine the length of time that the stent is positioned in the vascular before a therapeutic agent is delivered from the pores of the stent. Additionally, a therapeutic agent can be loaded into the bioabsorbable coating. Thus a therapeutic agent will be delivered to the stent at a rate determined by the bioabsorbability of the coating. Once the bioabsorbable material has completely dissolved, the therapeutic agent in the pores can be delivered at a rate determined by the pore size and porosity.

In another embodiment, it is preferred that the coating is permeable and non-absorbable. In such circumstances, the rate at which the drugs permeate into the tissue is controlled by the physical properties of the particular coating selected. Additionally, the coating may be selected to reduce restenosis, thrombosis or other tissue inflammation. For example, a heparin coating is known in the art to reduce blood clotting. Heparin, when coated on a stent, reduces clotting of blood on the surface of the stent. The heparin coating is affixed to the surface of the stent through ionic bonding, end-point attaching, or by photo-linking the heparin.

In yet another embodiment, a first therapeutic agent is loaded into the coating and a second therapeutic agent is loaded into the pores of the stent. This may be the case when a series of drug dosages or concentrations are needed. When such a stent is placed into the vasculature, the first therapeutic agent is absorbed first by the stent and a second therapeutic agent is absorbed later by the vasculature. This variation adds a further dimension to drug treatment allowing for sequential drug therapy at the site of placement of a stent.

## Claims

1. A stent comprising a body wherein at least a part of the body includes a first porous region (46) and a second porous region (44) disposed beneath the first porous region (46),
the second porous region (44) containing a therapeutic substance for the local delivery of the substance when the stent has been implanted in a vessel,
the average pore size of the first porous region (46) being less than the average pore size of the second porous region (44) for reducing the rate of release of the therapeutic substance from the second porous region (44), and
the first porous region (46) being formed by sintering metallic particles having a first diameter, and the second porous region (44) being formed by sintering metallic particles having a second diameter, the first diameter being smaller than the second diameter.

2. The stent of claim 1, additionally including a third porous region, such that the second porous region (44) is disposed between the first and third porous regions, wherein the average pore size of the third porous region is less than the average pore size of the second porous region (44).

3. The stent of claim 1, wherein the first porous region (46) is capable of contacting the inner wall of the vessel when the stent has been implanted in the vessel.

4. The stent of claim 1, additionally including a film layer disposed over the first porous region (46).

5. The stent of claim 4, wherein the film layer comprises a biodegradable polymer.

6. The stent of claim 4, wherein the film layer comprises a non-degradable polymer.

7. The stent of claim 4, wherein the film layer includes a second therapeutic substance.

8. The stent of claim 4, wherein the film layer includes heparin.

9. The stent of claim 1, wherein the therapeutic substance is for the treatment of restenosis.

10. The stent of claim 1, wherein the therapeutic substance comprises particles, and wherein the second porous region (44) includes pores (52) having an average pore size greater than ten times the particle size of the therapeutic substance.

11. The stent of claim 1, wherein the first or second porous region (46,44) is formed by sintering a matrix of overlapping metallic filaments.

12. The stent of claim 1, wherein the first or second porous region (46,44) is formed by sintering woven metallic wire fibers.

13. The stent of claim 1, wherein the first diameter is 2 to 4 µm.

14. The stent of claim 1, wherein the second diameter is 10 to 20 µm.

15. The stent of claim 1, wherein an inner core of a strut of the body defines the second porous region (44) and an outer layer surrounding the inner core defines the first porous region (46).

## Patentansprüche

1. Stent, der einen Körper umfasst, wobei zumindest ein Teil des Körpers einen ersten porösen Bereich (46) und einen zweiten porösen Bereich (44) aufweist, der unterhalb des ersten porösen Bereichs (46) angeordnet ist,
wobei der zweite poröse Bereich (44) eine therapeutische Substanz enthält, um die Substanz lokal zu verabreichen, wenn der Stent in ein Gefäß implantiert ist,
wobei die mittlere Porengröße des ersten porösen Bereichs (46) kleiner ist als die mittlere Porengröße des zweiten porösen Bereichs (44), um die Freisetzungsgeschwindigkeit der therapeutischen Substanz aus dem zweiten porösen Bereich (44) zu vermindern, und
wobei der erste poröse Bereich (46) aus gesinterten Metallpartikeln gebildet ist, die einen ersten Durchmesser aufweisen, und der zweite poröse Bereich (44) aus gesinterten Metallpartikeln gebildet ist, die einen zweiten Durchmesser aufweisen, wobei der erste Durchmesser kleiner als der zweite Durchmesser ist.

2. Stent nach Anspruch 1, der zusätzlich einen dritten porösen Bereich aufweist, sodass der zweite poröse Bereich (44) zwischen dem ersten und dem dritten porösen Bereich angeordnet ist, wobei die mittlere Porengröße des dritten porösen Bereichs kleiner ist als die mittlere Porengröße des zweiten porösen Bereichs (44).

3. Stent nach Anspruch 1, worin der erste poröse Bereich (46) ausgebildet ist, die Innenwand des Gefäßes zu berühren, wenn der Stent in das Gefäß implantiert ist.

4. Stent nach Anspruch 1, der zusätzlich eine dünne Schicht aufweist, die über dem ersten porösen Bereich (46) angeordnet ist.

5. Stent nach Anspruch 4, wobei die dünne Schicht ein biologisch abbaubares Polymer enthält.

6. Stent nach Anspruch 4, wobei die dünne Schicht ein nicht abbaubares Polymer enthält.

7. Stent nach Anspruch 4, wobei die dünne Schicht eine zweite therapeutische Substanz enthält.

8. Stent nach Anspruch 4, wobei die dünne Schicht Heparin enthält.

9. Stent nach Anspruch 1, wobei die therapeutische Substanz für die Behandlung von Restenose vorgesehen ist.

10. Stent nach Anspruch 1, wobei die therapeutische Substanz Partikel umfasst und wobei der zweite poröse Bereich (44) Poren (52) aufweist, die eine mittlere Porengröße von mehr als dem Zehnfachen der Partikelgröße der therapeutischen Substanz aufweisen.

11. Stent nach Anspruch 1, wobei der erste und zweite poröse Bereich (46, 44) durch Sintern einer Matrix von sich überlappenden Metallfilamenten gebildet ist.

12. Stent nach Anspruch 1, wobei der erste und zweite poröse Bereich (46, 44) durch Sintern gewebter Metalldrahtfasern gebildet ist.

13. Stent nach Anspruch 1, wobei der erste Durchmesser 2 bis 4 µm beträgt.

14. Stent nach Anspruch 1, wobei der zweite Durchmesser 10 bis 20 µm beträgt.

15. Stent nach Anspruch 1, wobei ein innerer Kern einer Stütze des Körpers den zweiten porösen Bereich (44) festlegt und eine äußere Schicht, die den inneren Kern umgibt, den ersten porösen Bereich (46) festlegt.

## Revendications

1. Endoprothèse comprenant un corps dans laquelle au moins une partie du corps comprend une première région poreuse (46) et une deuxième région poreuse (44) disposée en dessous de la première région poreuse (46),
la deuxième région poreuse (44) contenant une substance thérapeutique pour la délivrance locale de la substance lorsque l'endoprothèse a été implantée dans un vaisseau,
la taille de pore moyenne de la première région poreuse (46) étant plus petite que la taille de pore moyenne de la deuxième région poreuse (44) pour réduire le taux de libération de la substance thérapeutique de la deuxième région poreuse (44), et
la première région poreuse (46) étant réalisée par le frittage de particules métalliques ayant un premier diamètre, et la deuxième région poreuse (44) étant formée par le frittage de particules métalliques ayant un deuxième diamètre, le premier diamètre étant plus petit que le deuxième diamètre.

2. Endoprothèse selon la revendication 1, incluant additionnellement une troisième région poreuse, de telle sorte que la deuxième région poreuse (44) est disposée entre les première et troisième régions poreuses, où la taille de pore moyenne de la troisième région poreuse est plus petite que la taille de pore moyenne de la deuxième région poreuse (44).

3. Endoprothèse selon la revendication 1, dans laquelle la première région poreuse (46) est apte à venir en contact avec la paroi intérieure du vaisseau lorsque l'endoprothèse a été implanté dans le vaisseau.

4. Endoprothèse selon la revendication 1, incluant additionnellement une couche de film disposée sur la première région poreuse (46).

5. Endoprothèse selon la revendication 4, dans laquelle la couche de film comprend un polymère biodégradable.

6. Endoprothèse selon la revendication 4, dans laquelle la couche de film comprend un polymère non dégradable.

7. Endoprothèse selon la revendication 4, dans laquelle la couche de film comprend une deuxième substance thérapeutique.

8. Endoprothèse selon la revendication 4, dans laquelle la couche de film comprend de l'héparine.

9. Endoprothèse selon la revendication 1, dans laquelle la substance thérapeutique est pour le traitement de la resténose.

10. Endoprothèse selon la revendication 1, dans laquelle la substance thérapeutique comprend des particules, et où la deuxième région poreuse (44) comprend des pores (52) ayant une taille de pore moyenne plus grande que dix fois la taille de particule de la substance thérapeutique.

11. Endoprothèse selon la revendication 1, dans laquelle la première ou deuxième région poreuse (46, 44) est formée par le frittage d'une matrice de filaments métalliques qui se chevauchent.

12. Endoprothèse selon la revendication 1, dans laquelle la première ou deuxième région poreuse (46, 44) est formée par frittage de fibres de fil métallique tissées.

13. Endoprothèse selon la revendication 1, dans laquelle le premier diamètre est de 2 à 4 µm.

14. Endoprothèse selon la revendication 1, dans laquelle le deuxième diamètre est de 10 à 20 µm.

15. Endoprothèse selon la revendication 1, dans laquelle un noyau intérieur d'un tronçon du corps définit la deuxième région poreuse (44), et une couche extérieure entourant le noyau intérieur définit la première région poreuse (46).
